# EUROPÄISCHE PATENTSCHRIFT

(11) **EP 2 464 648 B1**
(45) Veröffentlichungstag und Bekanntmachung des Hinweises auf die Patenterteilung: **21.09.2016**
(21) Anmeldenummer: 10743109.0
(22) Anmeldetag: 12.08.2010
(51) Int. Cl.: C07D 487/04

(54) **Kristalline Verbindung von 7-[(3R)-3-Amino-1-oxo-4-(2,4,5-trifluorphenyl)butyl]-5,6,7,8-tetrahydro-3-(trifluormethyl)-1,2,4-triazolo[4,3-a]pyrazin**
Crystalline compound of 7-[(3R)-3-amino-1-oxo-4-(2,4,5-trifluorophenyl)butyl]-5,6,7,8-tetrahydro-3-(trifluoromethyl)-1,2,4-triazolo[4,3-a]pyrazine
Composé cristalline de 7-[(3R)-3-amino-1-oxo-4-(2,4,5-trifluorophényle)butyl]-5,6,7,8-tétrahydro-3-(trifluorométhyle)-1,2,4-triazolo[4,3-a]pyrazine

(30) Priorität: 13.08.2009 EP 09167825
(43) Veröffentlichungstag der Anmeldung: 20.06.2012
(73) Patentinhaber: Sandoz AG, 4056 Basel (CH)
(72) Erfinder: BLATTER, Fritz, CH-4153 Reinach (CH); REICHENBÄCHER, Katharina, CH-4125 Riehen (CH)
(74) Vertreter: Pommerenke, Alexander
(86) Internationale Anmeldenummer: PCT/EP2010/061733
(87) Internationale Veröffentlichungsnummer: WO 2011/018494

(56) Entgegenhaltungen:
- WO-A-03/004498
- WO-A-2005/003135
- WO-A-2005/072530
- WO-A-2009/085990
- CAIRA M R: "CRYSTALLINE POLYMORPHISM OF ORGANIC COMPOUNDS" TOPICS IN CURRENT CHEMISTRY, SPRINGER, BERLIN, DE, Bd. 198, 1. Januar 1998 (1998-01-01), Seiten 163-208, XP001156954
- KIM DOOSEOP ET AL: "(2R)-4-oxo-4-[3-(trifluoromethyl)-5,6-dih ydro[1,2,4]triazolo[4,3-a]py razin-7(8H)-yl]-1-(2,4,5-trifluorophenyl)b utan-2-amine: a potent, orally active dipeptidyl peptidase IV inhibitor for the treatment of type 2 diabetes." JOURNAL OF MEDICINAL CHEMISTRY 13 JAN 2005, Bd. 48, Nr. 1, 13. Januar 2005 (2005-01-13), Seiten 141-151, XP002557878 ISSN: 0022-2623 in der Anmeldung erwähnt

## Beschreibung

Die vorliegende Erfindung betrifft eine kristalline Verbindung von 7-[(3R)-3-Amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) mit Fumarsäure. Ein weiterer Gegenstand der vorliegenden Erfindung ist ein Verfahren zur Herstellung derselben.

Diabetes bezeichnet einen Erkrankungsprozess, der von mehreren ursächlichen Faktoren herrührt und sich durch erhöhte Plasmaglucosespiegel oder Hyperglykämie während des nüchternen Zustandes oder nach der Verabreichung von Glucose während eines oralen Glucosetoleranztests auszeichnet. Eine persistente oder nichtbekämpfte Hyperglykämie ist mit einer erhöhten und vorzeitigen Morbidität und Mortalität verbunden. Oft ist eine abnormale Glucosehomöostase sowohl direkt als auch indirekt mit Veränderungen des Lipid-, Lipoprotein-und Apolipoprotein-Stoffwechsels und mit anderen metabolischen und hämodynamischen Erkrankungen verbunden. Daher besteht für Patienten mit Typ-2-Diabetes mellitus ein besonders grosses Risiko für makrovaskuläre und mikrovaskuläre Komplikationen, einschliesslich koronarer Herzerkrankung, Apoplexie, peripherer vaskulärer Erkrankung, Hypertension, Nephropathie, Neuropathie und Retinopathie. Die therapeutische Bekämpfung von Glucosehomöostase, Lipidstoffwechsel und Hypertension ist für die klinische Handhabung und Behandlung von Diabetes mellitus von entscheidender Bedeutung.

Es existieren zwei allgemein anerkannte Formen von Diabetes. Bei der Typ-1-Diabetes oder insulinabhängigen Diabetes mellitus (IDDM) produzieren die Patienten wenig oder kein Insulin, ein Hormon, das die Glucoseverwertung steuert. Bei dem Typ-2-Diabetes oder nichtinsulinabhängigen Diabetes mellitus (NIDDM) besitzen die Patienten oft Plasmainsulinspiegel, die denen von nichtdiabetischen Patienten entsprechen oder sogar höher sind. Diese Patienten haben jedoch eine Resistenz gegenüber der insulinstimulierenden Wirkung auf den Glucose- und Lipidmetabolismus in den grossen insulinempfindlichen Geweben, welche Muskel-, Leber- und Fettgewebe sind, entwickelt, und die Plasmainsulinspiegel sind, obwohl erhöht, nicht ausreichend, um die ausgeprägte Insulinresistenz zu überwinden.

Die Insulinresistenz ist nicht in erster Linie die Folge einer verringerten Anzahl von Insulinrezeptoren, sondern ist die Folge eines Post Insulinrezeptorbindungsdefekts, der noch nicht aufgeklärt ist. Diese Resistenz gegenüber der Insulinempfindlichkeit führt zu einer ungenügenden Insulinaktivierung der Glucoseaufnahme, -oxidation und -speicherung im Muskel und zu einer unzureichenden Insulinhemmung der Lipolyse im Fettgewebe und der Glucoseerzeugung und -sekretion in der Leber.

Es hat sich gezeigt, dass es eine Vielzahl von Verbindungsklassen gibt, die zur Behandlung von Diabetes eingesetzt werden können. Besonders erwähnenswert sind hier die Inhibitoren des Dipeptidylpeptidase-IV-Enzyms ("DP-IV-Inhibitoren"), die sich zur Behandlung oder Prävention von Erkrankungen, bei denen das Dipeptidylpeptidase-IV-Enzym beteiligt ist, wie z.B. Diabetes und insbesondere Typ-2-Diabetes, eignen.

Die WO 03/004498 A1 schlägt als solche DP-IV-Inhibitoren Substanzen mit einer Pyrazin-Struktur vor, unter denen auch 7-[(3R)-3-Amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) genannt wird. Insbesondere wird auch ein Sitagliptin Hydrochlorid beschrieben. Dieses Hydrochlorid ist hygroskopisch.

In WO 2005/003135 wird ein Sitagliptin Dihydrogenphosphat Salz beschrieben, welches offensichtlich als Hydrat erhalten wurde, jedoch bei Temperaturen ab 40 °C phaseninstabil ist. Dies impliziert, dass die Herstellung von grösseren Mengen im technischen Massstab zu einem zu schwer kontrollierbaren Trocknungsprozess führt.

In der WO 2005/072530 A1 werden weitere Salze des Sitagliptins offenbart. Auch diese Verbindungen sollen als DP-IV-Inhibitoren zur Behandlung von Diabetes Verwendung finden. Weder die WO 03/004498 A1 noch die WO 2005/072530 A1 offenbaren Verbindungen enthaltend Sitagliptin und Fumarsäure.

Von Kim et al., J. Med. Chem 2005, 48, 141-151, werden oral aktive DP-IV-Inhibitoren zur Behandlung von Typ-2-Diabetes untersucht. Unter anderem wird Sitagliptin für eine in vivo Untersuchung mit Fumarsäure umgesetzt. Hierbei wird ein Feststoff erhalten, bei dem das Verhältnis von Sitagliptin zu Fumarsäure 1:0,5 ist. Eine Charakterisierung erfolgt nur durch NMR und HRMS. Es wird nicht angegeben, ob der erhaltene Feststoff kristallin ist.

In der WO2009/085990 A2 wird ein kristallines Fumarsäuresalz von Sitagliptin offenbart.

Im Stand der Technik besteht der Bedarf, Verbindungen enthaltend Sitagliptin bereitzustellen, die eine verbesserte Verabreichung ermöglichen. Insbesondere soll eine Darreichungsform von Sitagliptin zur Verfügung gestellt werden, die den Anforderungen an die Pharmakokinetik des Wirkstoffes gerecht wird und ausserdem in der Formulierung einer oralen Darreichungsform eingesetzt werden kann. Insbesondere soll eine Verbindung zur Verfügung gestellt werden, die eine optimierte niedrige Wasseraufnahme bei Lagerung aufweist.

Die technische Aufgabe der vorliegenden Erfindung wird durch eine kristalline Verbindung von 7-[(3R)-3-Amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) der Formel 1 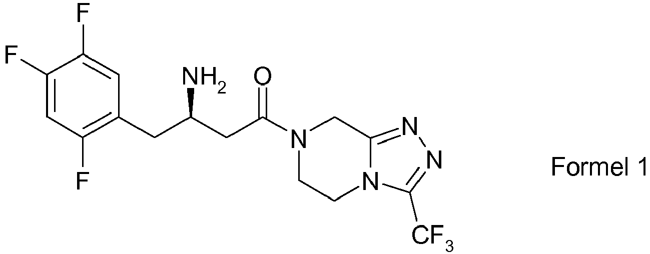 mit Fumarsäure, wobei das molare Verhältnis der Verbindung der Formel 1 zu Fumarsäure 1:0,95 bis 1:1,05 ist und die Verbindung mittels Röntgenpulverdiffraktometrie (XPRD) d-Werte (Å) bei 12.9 (w), 10.3 (w), 5.15 (m) und 4.30 (vs) aufweist, die nachfolgend als Form A bezeichnet wird gelöst.

Amorphe Formen sind in der Regel dadurch gekennzeichnet, dass diese keine scharfen Pulverdiffraktions-Reflexe zeigen. Das Pulverdiagramm von amorphen Formen besitzt lediglich ein stark erhöhtes Untergrundsignal im °2θ Bereich von ca. 10° bis 30°. Mesomorphe Formen sind normalerweise dadurch gekennzeichnet, dass diese nur sehr wenige, oft nur ein oder zwei, oder gar keine scharfen Pulverdiffraktions-Reflexe aufweisen. Mischungen von amorphen und kristallinen Phasen weisen vor allen dann nur sehr schwache Pulverdiffraktions-Reflexe auf, wenn der amorphe Anteil sehr hoch ist; zum Beispiel 50% oder höher.

Röntgenpulverdiffraktion ist eine weit verbreitete und anerkannte Methode zur Identifizierung und Charakterisierung von molekularen Festkörpern. Beschreibungen dieser Methode findet man sowohl in der Europäischen Pharmakopöe, als auch in der US Pharmacopeia als Methode Nr. 941 "X-Ray Diffraction", oder in "Polymorphism - In the Pharmaceutical Industry" Kapitel 6, Rolf Hilfiker, Editor Wiley-VCH Verlag, Weinheim, Deutschland, 2006. Normalerweise wird Röngten-Pulverdiffraktion mit Kupfer-Kα Strahlung durchgeführt, wie dies auch hier der Fall war. D-Werte in 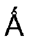 (d) und 2-theta (2θ) Werte in Winkelgrad lassen sich durch die Bragg'sche Gleichung wie folgt ineinander umrechnen: nλ = 2dsinθ, wobei n eine ganze Zahl und λ die Wellenlänge der verwendeten Röntgenstrahlung in 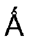 ist. Die aus einer Pulverdiffraktionsmessung erhaltenen Daten sind Signalintensität (in Counts) in Abhängigkeit des Winkels 2θ. Im Weiteren ist zu beachten, dass sowohl in Reflexionsgeometrie, als auch in Transmissionsgeometrie gemessen werden kann. Die hier erwähnten Messungen wurden in Reflexions-geometrie ausgeführt. Während die Lage der Linien in Winkelgrad nicht von der Geometrie abhängig ist, können sich aber die relativen Intensitäten sowohl durch die Geometrie, als auch durch die Eigenschaften der Probe und durch die Probenpräparation ändern. Deshalb dienen die angegeben Intensitäten nur als qualitatives Merkmal. Bei Messungen dieser Art beträgt der Messfehler üblicherweise ±0,1°2θ. Während sich der Messfehler für °2θ über den gesamten Messbereich nur geringfügig oder gar nicht verändert, ist aufgrund der oben erwähnten Bragg'schen Gleichung der Fehler bei den d-Werten abhängig vom Winkel. 2θ Winkel und d-Werte sind aber äquivalent und deshalb ist für die d-Werte kein Messfehler ausgerechnet worden, obwohl ein solcher vorliegt.

Eine Besonderheit der hier vorliegenden Erfindung sind kristalline Verbindungen von Sitagliptin mit Fumarsäure, welche sich durch eine hohe kristalline Reinheit hervorheben. Dies ist dadurch gekennzeichnet, dass die Pulverröntgendiagramme dieser Verbindungen mindestens vier Peaks aufweisen, von denen jeder vorzugsweise ein Signal zu Rausch Verhältnis von mindestens 4 aufweist. Weiter bevorzugt ist ein Signal zu Rausch Verhältnis von mindestens 8 oder höher, wobei die Röntgenpulverdiffraktionsmessungen unter den hier beschriebenen oder äquivalenten Messbedingungen durchzuführen sind.

Im Vergleich zum bekannten Stand der Technik besitzt - in Bezug auf Verbindungen enthaltend Fumarsäure - die kristalline Verbindung gemäss dieser Erfindung vorzugsweise eine grosse Anzahl und scharfe Pulverdiffraktions-Reflexe.

Raman Spektroskopie ist eine zweite sehr nützliche Methode zur Identifizierung und Charakterisierung verschiedener Formen von molekularen Festkörpern. Detailliertere Beschreibungen der Anwendung von Raman Spektroskopie zum erwähnten Zweck findet man z.B. in "Polymorphism - In the Pharmaceutical Industry" Kapitel 5, Rolf Hilfiker, Editor Wiley-VCH Verlag, Weinheim, Deutschland, 2006. Bei Messungen dieser Art beträgt der Messfehler üblicherweise ±1 cm ⁻¹.

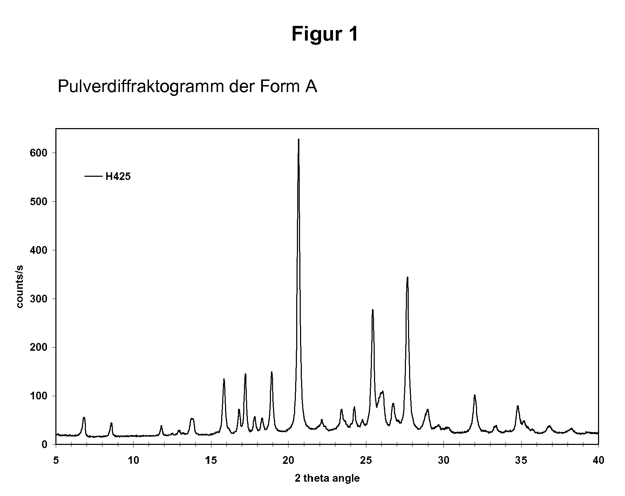

In der vorliegenden Erfindung weist die kristalline Verbindung mittels Röntgenpulverdiffraktometrie (XRPD) d-Werte (A) bei 12.9 (w), 10.3 (w), 5.15 (m) und 4.30 (vs) auf, die nachfolgend als Form A bezeichnet wird. Weiter bevorzugt weist die Verbindung (Form A) mittels Röntgenpulverdiffraktometrie (XRPD) d-Werte (A) bei 12.9 (w), 10.3 (w), 5.15 (m), 4.69 (m), 4.30 (vs), 3.50 (s), 3.22 (s) auf. Figur 1 zeigt ein Pulverdiffraktogramm der Form A.

Das molare Verhältnis der Verbindung der Formel 1 zu Fumarsäure ist 1:0,95 bis 1:1,05, wobei dieses Verhältnis bei der Form A vorliegt.

Die Form A weist vorzugsweise charakteristische Raman-Banden bei 3083, 3034, 3019, 2940, 1707, 1655, 1516, 1479,1439, 1389, 1338, 1260, 979, 899, 807, 756, 748, 697, 537, 453, 396, 288, 213, 104 und 85 cm⁻¹ (Wellenzahlen) auf. Die intensiveren Raman-Banden der Form A findet man vorzugsweise bei 3034, 2940, 1655, 1516, 756, und 104 cm⁻¹ (Wellenzahlen).

Amorphe oder mesomorphe Formen einer Verbindung können viel stärker zur Hygroskopie neigen als kristalline Formen. Die hier beschriebenen Sitagliptin Fumarat Verbindungen zeichnen sich dadurch aus, dass sie gute hygroskopische Eigenschaften besitzen, d.h. bei hoher relativer Feuchte wenig Wasser aufnehmen. Dies ist aus der nachfolgenden Tabelle 1 ersichtlich, welche die Wassergehalte vom kristallinen Fumarat Form A und kristallinen Hydrochlorid bei 50% und 90% relativer Feuchte vergleicht. Die Daten stammen aus dynamischen Wasserdampfadsorptionsmessungen. Die Wasserdampfsorptionsmessung ist eine geeignete Methode, um die hygroskopischen Eigenschaften von festen Substanzen zu untersuchen. Wasserdampfsorptionsmessungen können auf verschiedene Arten durchgeführt werden. Im Allgemeinen wird dabei eine kleine Probe von ca. 10 - 30 mg in einem geeigneten Probenträger in eine Mikrowaage gebracht. Die Probe wird dann gemäss einem definierten Programm verschiedenen relativen Feuchten ausgesetzt, wobei gleichzeitig die Massenänderung der Probe über die Zeit aufgenommnen wird. Als Resultat können so Einblicke in das hygroskopische Verhalten einer Substanz erhalten werden. Sowohl kristallines Sitagliptin Hydrochlorid Monohydrat als auch kristalline Verbindungen der vorliegenden Erfindung wurden mit dieser Methode untersucht und dabei wurde festgestellt, dass das Hydrochlorid unter gleichen Messbedingungen signifikant mehr Wasser adsorbiert und somit stärker hygroskopisch ist. Es hat sich gezeigt, dass beispielsweise kristallines Fumarat in der Form A bei 50% relativer Feuchte nur ca. 0.3% Wasser enthält und nach vier Stunden bei 96% relativer Feuchte nur gerade ca. 1% mehr Wasser aufnimmt als bei 50% relativer Feuchte, wobei letzterer Wert in etwa den normalen Feuchtigkeitsbedingungen in Mitteleuropa entspricht.

**Tabelle 1: Ergebnisse der Wasserdampfsorptionsmessungen**

| Salzform | H₂O Gehalt bei 50% r.h. | H₂O Gehalt bei 90% r.h. |
|---|---|---|
| kristallines Sitagliptin Fumarat Form A | 0.25% | 1.33% |
| kristallines Sitagliptin Hydrochlorid gemäss Stand der Technik | >2.7% | >3.6% |

Dieses Ergebnis ist für den Fachmann unerwartet und aus dem Stand der Technik nicht herleitbar. Folglich bieten die kristallinen Verbindungen der vorliegenden Erfindung ein Eigenschaftsprofil, das vorteilhaft zur Verwendung in Medikamenten ist.

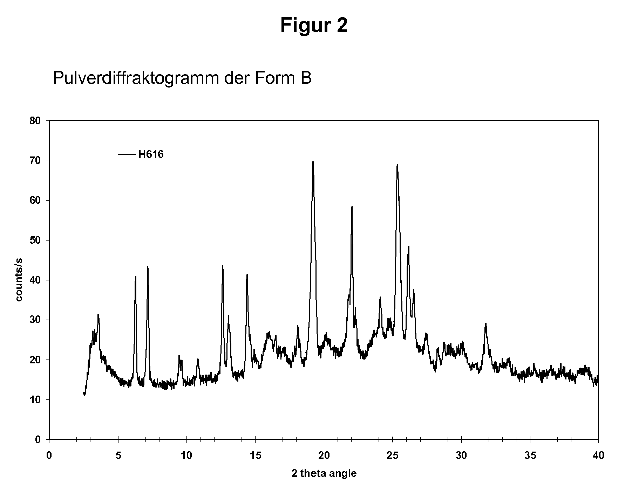

In einer weiteren hier beschriebenen Form weist die kristalline Verbindung mittels Röntgenpulverdiffraktometrie (XRPD) d-Werte (A) bei 12.3 (s), 7.0 (s), 4.62 (vs) und 3.51 (vs) auf, die nachfolgend als Form B bezeichnet wird. In Form B weist die Verbindung mittels Röntgenpulverdiffraktometrie (XRPD) d-Werte bei 14.1 (s), 12.3 (s), 7.0 (s), 6.1 (s), 4.62 (vs), 4.03 (vs) und 3.51 (vs) auf. Figur 2 zeigt ein Pulverdiffraktogramm der Form B.

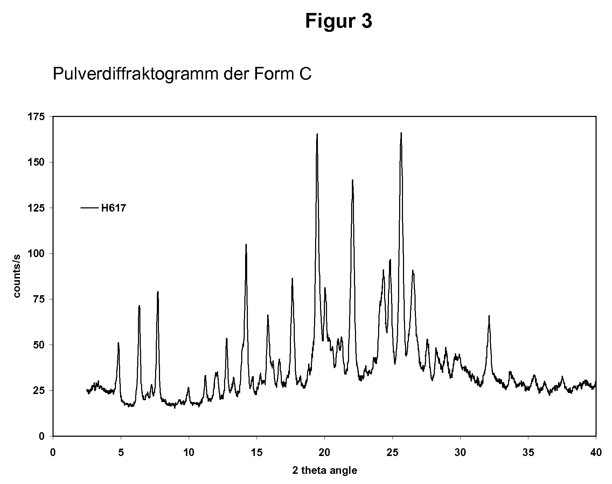

In einer weiteren hier beschriebenen Form weist die Verbindung mittels Röntgenpulverdiffraktometrie (XRPD) d-Werte (A) bei 18.3 (m), 11.4 (s), 4.56 (vs) und 3.47 (vs) auf, die nachfolgend als Form C bezeichnet wird. In Form C weist die Verbindung mittels Röntgenpulverdiffraktometrie (XRPD) d-Werte bei 18.3 (m), 13.9 (s), 12.2 (w), 11.4 (s), 6.2 (s), 5,02 (m), 4.56 (vs), 4.03 (vs) und 3.47 (vs) auf. Figur 3 zeigt ein Pulverdiffraktogramm der Form C.

Ein weiterer Gegenstand der vorliegenden Erfindung ist ein Medikament, enthaltend die kristalline Verbindung in Form A.

Die kristalline Verbindung in Form A wird vorzugsweise zur Behandlung von Diabetes eingesetzt.

Ein weiterer Gegenstand der vorliegenden Erfindung ist ein Verfahren zur Herstellung der kristallinen Verbindung in Form A.

Das Verfahren umfasst die Schritte:
a) Bereitstellung einer Lösung von 7-[(3R)-3-Amino-1-oxo-4-(2,4,5-trifluoro-phenyl)butyl]-5,6,7,8-tetrahydro-3-(trifluoromethyl)-1,2,4-triazolo[4,3-a]pyrazine (INN: Sitagliptin) der Formel 1,
b) Zugabe von Fumarsäure zu der Lösung des Schrittes a),
c) optionales Aufkonzentrieren der nach Schritt b) erhaltenen Zusammensetzung und/oder optionale Zugabe eines geeigneten Nichtlösungsmittels zur Erniedrigung der Löslichkeit der kristallinen Verbindung, und
d) Abtrennung und anschliessende Trocknung des erhaltenen Feststoffes.

Vorzugsweise kann die Fumarsäure in Schritt b) als Feststoff zu der Lösung des Schrittes a) gegeben werden. Alternativ kann die Fumarsäure des Schrittes b) als eine Lösung in einem geeigneten Lösungsmittel zu der Lösung des Schrittes a) gegeben werden. In einer weiter bevorzugten Ausführungsform ist es auch möglich, dass die Schritte a) und b) vertauscht werden, also die Lösung des Schrittes a) enthaltend Sitagliptin zu Fumarsäure des Schrittes b) gegeben wird.

Vorzugsweise ist das Lösungsmittel des Schrittes a) und/oder optionales Lösungsmittel des Schrittes b) jeweils ein organisches oder anorganisches Lösungsmittel.

In einer bevorzugten Ausführungsform ist das molare Verhältnis der gelösten Verbindung der Formel 1 in Schritt a) zu der Fumarsäure in Schritt b) 1:0,6 bis 1:1,3.

Es ist weiter bevorzugt, dass zur Herstellung der Form A das molare Verhältnis der gelösten Verbindung der Formel 1 in Schritt a) zu der Fumarsäure in Schritt b) 1:0,90 bis 1:1,10 und weiter bevorzugt 1:0,95 bis 1:1,05 ist.

Es ist ausserdem bevorzugt, dass zur Herstellung der Form B oder C das molare Verhältnis der gelösten Verbindung der Formel 1 in Schritt a) zu der Fumarsäure in Schritt b) 1:0,60 bis 1:0,80 ist.

Weiter bevorzugt werden für den Schritt a) und/oder Schritt b) niedermolekulare und physiologisch verträgliche Lösungsmittel und/oder physiologisch verträgliche Alkohole, wie z.B. Ethanol, Isopropanol, 1-Propanol, n-Butanol, niedermolekulare Ketone, wie z. B. Aceton, 2-Butanon, Methyl-Isobutyl Keton, Acetate, z.B. Ethyl Formiat, Ethyl Acetate, Butyl Acetat oder Isopropyl Acetat oder Ether, z.B. tert-Butyl Methyl Ether, Diethylether, Diisopropylether oder beliebig zu kombinierende Mischungen hiervon eingesetzt. Besonders bevorzugt für den Schritt a) und/oder Schritt b) sind physiologisch verträgliche Lösungsmittel in welchen die Fumarsäure in genügender Weise löslich ist. Bevorzugt sind Ethanol, 1-Propanol, 2-Propanol, THF, Aceton, Ethylmethyl Keton, Wasser und Mischungen derselben. Besonders bevorzugt für Schritt a) sind Ethanol, 2-Propanol, Ethyl Acetat, Isopropyl Acetat, Aceton, Ethylmethyl Keton und für Schritt b) Ethanol, 2-Propanol, Aceton, Ethylmethyl Keton und Wasser.

Mit physiologisch verträglich ist gemeint, dass diese Lösungsmittel unter die ICH (International Commission for Harmonization) Richtline Q3C, Klasse 3, fallen. In der Reihenfolge können die Schritte a) und b) vertauscht werden.

In den Schritten a) und b) können die beiden Komponenten sowohl in gleichen, als auch in unterschiedlichen Lösungsmitteln, und sowohl in gleichen, als auch in unterschiedlichen Konzentrationen gelöst werden. Es ist auch möglich, jeweils Lösungsmittelgemische aus zwei oder mehr Lösungsmitteln im Schritt a) und/oder Schritt b) einzusetzen.

Alternativ ist es bevorzugt, eine Verbindung von 7-[(3R)-3-Amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) der Formel 1 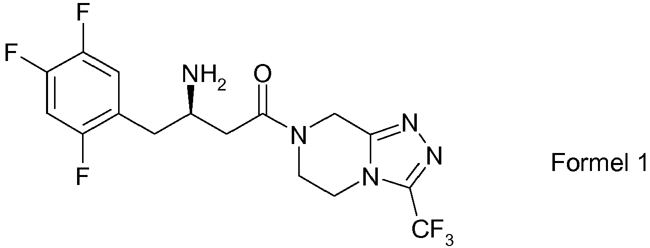 mit Fumarsäure oder ein Hydrat hiervon als Ausgangsmaterial für die Kristallisation einzusetzen.

In Schritt c) können vorzugsweise Impfkristalle der gewünschten Form zugegeben werden. In Schritt c) ist es weiter bevorzugt, dass die erhaltene Suspension bei einer geeigneten Temperatur gerührt wird. Vorzugsweise liegt diese Temperatur im Bereich von 5 °C bis 50 °C.

Weiter kann in den Schritten a) und b) die Kristallisationstemperatur, respektive das Erreichen der Sättigung bezüglich des gewünschten Salzes durch die Auswahl verschiedener Kombinationen der Lösungsmittel und Konzentrationen so moduliert werden, dass das gewünschte Salz in hoher Ausbeute und hoher Reinheit erhalten wird.

Zur Herstellung der Form A wird vorzugsweise Sitagliptin und Fumarsäure in 2-Butanon (Methyethylketon) oder Acetonitril gerührt. Das molare Verhältnis von Sitagliptin zu Fumarsäure ist hierbei vorzugsweise 1:1 ± 0,1. Vorzugsweise wird die Zusammensetzung für mindestens einen Tag bei vorzugsweise Raumtemperatur gerührt. In einer weiter bevorzugten Ausführungsform wird das Lösungsmittel zumindest teilweise während des Rührens eingeengt.

Ein hier beschriebenes Verfahren zur Herstellung der Form B umfasst das Umkristallisieren der Form A aus einer Lösungsmittelmischung von Ethanol und Essigsäureethylester.

Vorzugsweise kann die Form B erhalten werden, indem man die Form A als Slurry in einer Mischung aus Ethanol und Wasser rührt. Vorzugsweise wird der Slurry für mindestens einen Tag bei vorzugsweise Raumtemperatur gerührt.

Ein hier beschriebenes Verfahren zur Herstellung der Form C umfasst das Umkristallisieren der Form A aus dem Lösungsmittel 2-Propanol. Überraschenderweise kann Form A als Ausgangsmaterial zur Herstellung weiterer Formen, wie Form B oder Form C der kristallinen Verbindung der vorliegenden Erfindung eingesetzt werden. Form A ist in hoher Reinheit erhältlich. Hierbei wird Form A der vorliegenden Erfindung vorzugsweise in einem geeigneten Lösungsmittel oder Lösungsmittelgemisch vorgelegt. Nachfolgend erhält man durch Umkristallisation die gewünschte Form der kristallinen Verbindung der vorliegenden Erfindung, wobei vorzugsweise Impfkristalle der gewünschten Form, wie Form B oder Form C, zugegeben werden. Als Lösungsmittel oder Lösungsmittelgemisch können alle vorgenannten Lösungsmittel verwendet werden. Besonders bevorzugt sind Ethanol, Essigsäureethylester, 2-Propanol und Mischungen derselben. Optional kann eine geeignete Menge Wasser zugegeben werden.

### Beispiele

### Röntgenpulverdiffraktion

Pulverdiffraktogramme wurden auf einem Bruker D8 Advance Pulverdiffraktometer mit einem Goniometerradius von 217.5 mm unter Verwendung von Kupfer Kα Strahlung gemessen. Dieses Gerät arbeitet in Reflektions Bragg-Brentano Geometrie bei einer Anodenspannung von 40 kV und einem Strom von 40 mA, wobei eine variable Divergenzblende verwendet wurde. Das Bruker D8 Gerät ist ausgerüstet mit einem LynxEye Detektor, wobei das aktive Beobachtungsfenster auf 3° eingestellt wurde. Die Schrittweite betrug 0.02° und die äquivalente Akkumulationszeit 37 Sekunden pro Schritt. Die Proben wurden ohne weitere Behandlung auf zirkulären Silizium-Einkristall Trägern mit einer Tiefe von 0.1 mm und einem Durchmesser von 12 mm präpariert. Die Proben wurden während der Messung mit 0.5 Drehungen pro Sekunde rotiert. Der Messfehler beträgt etwa ±0,1°2θ.

### Raman Spektroskopie

Fourier Transformation Raman Spektroskopie wurde mit einem Bruker RFS100 durchgeführt, welches zur Anregung einen Nd:YAG Laser mit einer Wellenlänge von 1064 nm besitzt. Die verwendete Laserleistung war 300 mW. Das verwendete Gerät ist mit einem Flüssigstickstoff gekühlten Germanium-Detektor ausgerüstet. Ca. 3 mg der Messprobe werden in einen kleinen Aluminiumträger gepresst und dieser Träger wird in die Spektrometer-Messkammer eingesetzt. Zur Aufnahme von Spektren wurden 64 Akkumulationen mit einer Auflösung von 2.0 cm⁻¹ im Bereich von 100-3500 cm⁻¹ Wellenzahlen gemessen. Der Messfehler beträgt etwa ±1 cm⁻¹.

### Wasserdampfadsorptionsmessungen

Dynamische Wasserdampfadsorptionsmessungen wurden mit einem SPS11-100n Gerät, hergestellt von der Firma "Projekt Messtechnik" in Ulm, Deutschland, durchgeführt. Dazu wurden ca. 20 mg der Probe in einen Aluminiumträger eingewogen und dieser in der Messkammer des Geräts eingesetzt. Die Probe wurde dann gemäss einem definierten Programm vorgewählten relativen Feuchten ausgesetzt, wobei die Massenänderung über die Zeit bestimmt wird. Das folgende Messprogramm wurde verwendet: 50% r.h. konstant während zwei Stunden, dann Änderung der relativen Feuchte auf 0% r.h., anschliessend Änderung der relativen Feuchte auf 96% r.h., gleichbleibend 96% r.h. während vier Stunden und dann Änderung der relativen Feuchte auf 50% r.h. und dann gleichbleibend 50% r.h. während einer Stunde. Die eingestellten Änderungsraten waren jeweils 5% pro Stunde.

¹H-NMR Spektroskopie

¹H-NMR Spektroskopie wurde auf einem Bruker DPX300 Gerät durchgeführt. Sitagliptin Fumarat Proben wurden in der Regel in DMSO- d₆ gemessen.

### Herstellung der kristallinen Sitagliptin Fumarat Verbindungen

### Beispiel 1

500.7 mg (1.23 mmol) Sitagliptin freie Base und 142.8 mg (1.23 mmol) Fumarsäure wurden in 50 ml Ethylmethylketon bei Raumtemperatur gerührt. Nach einer Stunde wurde die erhaltene klare Lösung mittels Heissluftföhn erhitzt und im Ultraschallbad abgekühlt. Einengen der Lösung auf ca. 40 ml durch N₂-Strom führte zu einer Fällung, welche filtriert wurde. Zur Mutterlauge wurden Impfkristalle der ersten Fällung zugegeben und bei Raumtemperatur gelagert. Die erhaltene Suspension wurde filtriert und der weisse Feststoff 30 min bei etwa 3 mbar getrocknet. Mittels Röntgenpulverdiffration findet man das typische Pulverdiagramm der Form A, welches in der Figur 1 abgebildet ist. Die wichtigsten Reflexe sind in untenstehender Tabelle 2 erwähnt. Die ¹H-NMR Spektroskopie derselben Probe zeigt ein molares Verhältnis von Sitagliptin zu Fumarsäure von 1:1.

**Tabelle 2: Pulverröntgen Daten für die Form A**

| °2θ Winkel | d-Wert [Ǻ] | relative Intensitäten (qualitativ) |
|---|---|---|
| 6.8 | 12.9 | W |
| 8.6 | 10.3 | W |
| 15.8 | 5.59 | m |
| 17.2 | 5.15 | m |
| 18.9 | 4.69 | m |
| 20.7 | 4.30 | vs |
| 25.4 | 3.50 | s |
| 27.7 | 3.22 | s |

| | | |
|---|---|---|
| w steht für weak, m für medium, s für strong und vs für very strong | | |

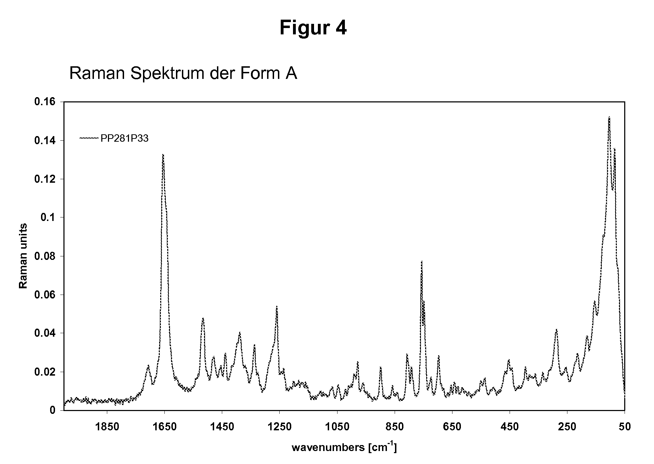

Die Raman-spektroskopische Untersuchung des erhaltenen Produktes zeigt das charakteristische Raman Spektrum der Form A, welches in Figur 4 abgebildet ist. Die wichtigsten Peaks sind in Tabelle 3 angegeben.

**Tabelle 3: Raman Spektrum der Form A**

| Wellenzahl [cm⁻¹] | relative Intensität (qualitativ) |
|---|---|
| 3083 | m |
| 3034 | m |
| 3019 | m |
| 2940 | s |
| 1707 | w |
| 1655 | vs |
| 1516 | m |
| 1479 | w |
| 1439 | w |
| 1389 | m |
| 1338 | m |
| 1260 | m |
| 979 | w |
| 899 | w |
| 807 | w |
| 791 | w |
| 756 | s |
| 748 | m |
| 697 | w |
| 537 | w |
| 453 | w |
| 396 | w |
| 288 | m |
| 181 | m |
| 104 | vs |
| 85 | vs |

| | |
|---|---|
| w steht für weak, m für medium, s für strong und vs für very strong | |

### Beispiel 2

2.0 g (4.91 mmol) Sitagliptin freie Base und 571 mg (4.92 mmol) Fumarsäure wurden in 150 ml Ethylmethylketon bei Raumtemperatur für 1 h gerührt. Nach Zugabe von weiteren 60 ml Ethylmethylketon wurde die trübe Lösung mittels Heissluftföhn erhitzt. Nach Abkühlen auf Raumtemperatur wurde der nun klaren Lösung Impfkristalle des Sitagliptin Fumarat zugesetzt und die Lösung bei Raumtemperatur unter Lichtausschluss gelagert. Nach drei Tagen wurde die erhaltene Suspension filtriert und der weisse Feststoff 2 Stunden bei etwa 3 mbar getrocknet (Ausbeute: 1.35 g). Das erhaltene Material wurde mittels Raman Spektroskopie, ¹H-NMR Spektroskopie, Elementaranalyse, Thermogravimetrie, gekoppelt mit Infrarot Spektroskopie, sowie mit dynamischer Wasserdampfsorptionsmessung charakterisiert. Die Thermogravimetrie gekoppelt mit FT-IR Spetkroskopie zeigt, dass die minimal getrocknete Probe nur gerade ca. 0.5% Wasser sowie eine geringe Menge Ethylmethyl Keton enthält. ¹H-NMR Spektroskopie zeigt ein Verhältnis von Sitagliptin zu Fumarsäure von 1:1. Unter Berücksichtigung der kleinen Gehalte an Wasser und Restlösungsmittel ist dieses Verhältnis in Übereinstimmung mit dem Resultat der Elementaranalyse, welche die folgenden Werte ergab: C = 45.5%, H = 3.5%, N = 13.2%, O = 15.6% und F = 21.6%. Die Raman Spektroskopie zeigt das typische Raman Spektrum der Form A. Die dynamische Wasserdampfsorption zeigt, dass die Form A, selbst bei hohen relativen Feuchten, nur wenig Wasser aufnimmt.

Vergleichsbeispiel 3 67.5 mg Sitagliptin Fumarat aus Beispiel 2 wurde in 4 ml Ethanol/Ethylacetat (1:1 v/v) bei 65°C gelöst. Die klare Lösung wurde auf 20°C mit 0.5°C/h abgekühlt, wobei eine Suspension gebildet wurde. Nach Filtration wurde der weisse Feststoff 15 Stunden bei 3 mbar getrocknet. Das erhaltene Produkt wurde mittels Raman Spektroskopie, Röntgenpulverdiffraktion sowie ¹H-NMR Spektroskopie untersucht. Die ¹H-NMR Spektroskopie zeigt ein molares Verhältnis von Sitagliptin zu Fumarsäure von 1:0,8. Raman Spektroskopie und Röntgenpulverdiffraktion zeigen das charakteristische Spektrum respektive Diffraktogramm, welche für Sitagliptin Form B typisch sind.

Figur 2 zeigt ein Pulverdiffraktogramm der Form B. Die wichtigsten Reflexe sind in untenstehender Tabelle 4 erwähnt.

**Tabelle 4: Pulverröntgen Daten für die Form B**

| °2θ Winkel | d-Wert [Ǻ] | relative Intensitäten (qualitativ) |
|---|---|---|
| 6.2 | 14.1 | s |
| 7.2 | 12.3 | s |
| 12.6 | 7.0 | s |
| 14.4 | 6.1 | s |
| 19.2 | 4.62 | vs |
| 22.0 | 4.03 | vs |
| 25.3 | 3.51 | vs |

| | | |
|---|---|---|
| w steht für weak, m für medium, s für strong und vs für very strong | | |

Vergleichsbeispiel 4 46.3 mg Sitagliptin Fumarat aus Beispiel 2 wurde in 12.4 ml 2-Propanol bei 65°C gelöst. Die klare Lösung wurde auf 20°C mit 0.5°C/h abgekühlt. Da keine Fällung zu beobachten war, wurde die Lösung 2.5 Stunden bei 5°C gerührt. Die gebildete gelartige Suspension wurde 7 Tage bei Raumtemperatur gerührt und anschliessend filtriert. Der Feststoff wurde 15 Stunden bei etwa 3 mbar getrocknet. Das erhaltene Produkt wurde mittels Raman Spektroskopie, Röntgenpulverdiffraktion sowie ¹H-NMR Spektroskopie untersucht. Die ¹H-NMR Spektroskopie zeigt ein molares Verhältins von Sitagliptin zu Fumarsäure von 1:0,7. Raman Spektroskopie und Röntgenpulverdiffraktion zeigen das charakteristische Spektrum respektive Diffraktogramm, welche für Sitagliptin Form C typisch sind.

Figur 3 zeigt ein Pulverdiffraktogramm der Form C. Die wichtigsten Reflexe sind in untenstehender Tabelle 5 erwähnt.

**Tabelle 5: Pulverröntgen Daten für die Form C**

| °2θ Winkel | d-Wert [Ǻ] | relative Intensitäten (qualitativ) |
|---|---|---|
| 4.8 | 18.3 | m |
| 6.4 | 13.9 | s |
| 7.3 | 12.2 | w |
| 7.7 | 11.4 | s |
| 14.2 | 6.2 | s |
| 17.7 | 5.02 | m |
| 19.4 | 4.56 | vs |
| 22.1 | 4.03 | vs |
| 25.6 | 3.47 | vs |

| | | |
|---|---|---|
| w steht für weak, m für medium, s für strong und vs für very strong | | |

## Patentansprüche

1. Kristalline Verbindung von 7-[(3R)-3-Amino-1-oxo-4-(2,4,5-trifluoro-phenyl)butyl]-5,6,7,8-tetrahydro-3-(trifluoromethyl)-1,2,4-triazolo[4,3-a]pyrazine (INN: Sitagliptin) der Formel 1 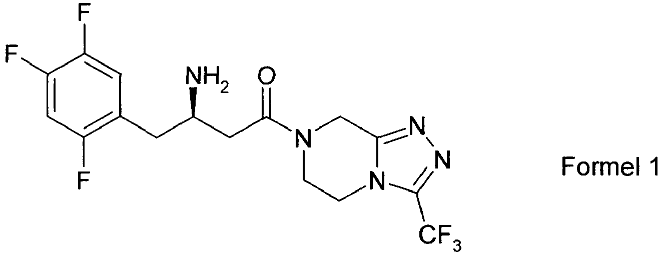 mit Fumarsäure, wobei das molare Verhältnis der Verbindung der Formel 1 zu Fumarsäure 1:0,95 bis 1:1,05 ist und die Verbindung mittels Röntgenpulverdiffraktometrie (XRPD) d-Werte (A) bei 12.9 (w), 10.3 (w), 5.15 (m) und 4.30 (vs) aufweist, die nachfolgend als Form A bezeichnet wird.

2. Die kristalline Verbindung gemäss Anspruch 1, **dadurch gekennzeichnet, dass** die Verbindung mittels Röntgenpulverdiffraktometrie (XRPD) d-Werte (A) bei 12.9 (w), 10.3 (w), 5.15 (m), 4.69 (m), 4.30 (vs), 3.50 (s), 3.22 (s) aufweist.

3. Die kristalline Verbindung gemäss wenigstens einem der Ansprüche 1 und 2 für die Verwendung in der Behandlung von Diabetes.

4. Ein Medikament enthaltend die kristalline Verbindung gemäss wenigstens einem der vorhergehenden Ansprüche.

5. Die Verwendung der kristallinen Verbindung gemäss wenigstens einem der Ansprüche 1 bis 3 zur Herstellung eines Medikamentes zur Behandlung von Diabetes.

6. Verfahren zur Herstellung der Verbindung gemäss wenigstens einem der Ansprüche 1 bis 2 umfassend die Schritte:
a) Bereitstellung einer Lösung von 7-[(3R)-3-Amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) der Formel 1,
b) Zugabe von Fumarsäure zu der Lösung des Schrittes a),
c) optionales Aufkonzentrieren der nach Schritt b) erhaltenen Zusammensetzung und/oder optionale Zugabe eines geeigneten Nichtlösungsmittels zur Erniedrigung der Löslichkeit der kristallinen Verbindung, und
d) Abtrennung des erhaltenen Feststoffes.

7. Das Verfahren gemäss Anspruch 6, **dadurch gekennzeichnet, dass** das molare Verhältnis der gelösten Verbindung der Formel 1 in Schritt a) zu der Fumarsäure in Schritt b) 1:0,6 bis 1:1,3 ist.

8. Das Verfahren gemäss Anspruch 6 oder 7, **dadurch gekennzeichnet, dass** die Fumarsäure in Schritt b) als Feststoff zu der Lösung des Schrittes a) gegeben wird oder die Fumarsäure des Schrittes b) als eine Lösung in einem geeigneten Lösungsmittel zu der Lösung des Schrittes a) gegeben wird.

9. Verfahren gemäß wenigstens einem der Ansprüche 6 bis 8 zur Herstellung der Form A, wobei die kristalline Verbindung der Formel 1 (Sitagliptin) und Fumarsäure in 2-Butanon (Methylethylketon) gerührt werden und das molare Verhältnis von Sitagliptin zu Fumarsäure vorzugsweise 1:1 ± 0,1 ist.

## Claims

1. Crystalline compound of 7-[(3R)-3-amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) of formula 1 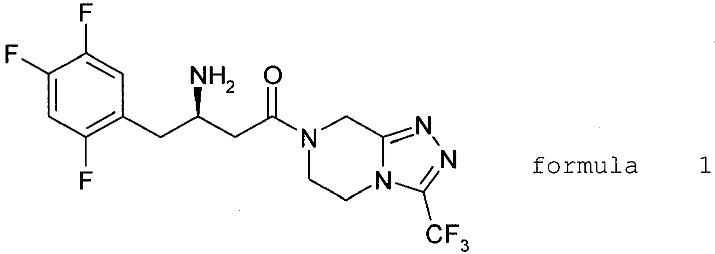 with fumaric acid, where the molar ratio of the compound of formula 1 to fumaric acid is 1:0.95 to 1:1.05 and the compound has, by means of X-ray powder diffractometry (XRPD) d values (Å) at 12.9 (w), 10.3 (w), 5.15 (m) and 4.30 (vs), which is subsequently referred to as form A.

2. Crystalline compound according to Claim 1, **characterized in that** the compound has, by means of X-ray powder diffractometry (XRPD) d values (Å) at 12.9 (w), 10.3 (w), 5.15 (m), 4.69 (m), 4.30 (vs), 3.50 (s), 3.22 (s).

3. Crystalline compound according to at least one of Claims 1 and 2 for the use in the treatment of diabetes.

4. Medicament comprising the crystalline compound according to at least one of the preceding claims.

5. Use of the crystalline compound according to at least one of Claims 1 to 3 for producing a medicament for the treatment of diabetes.

6. Method for producing the compound according to at least one of Claims 1 to 2, comprising the steps:
a) provision of a solution of 7-[(3R)-3-amino-1-oxo-4-(2,4,5-trifluorophenyl)butyl]-5,6,7,8-tetrahydro-3-(trifluoromethyl)-1,2,4-triazolo[4,3-a]pyrazine (INN: Sitagliptin) of formula 1,
b) addition of fumaric acid to the solution in step a),
c) optional concentration of the composition obtained after step b) and/or optional addition of a suitable non-solvent for lowering the solubility of the crystalline compound, and
d) removal of the solid obtained.

7. Method according to Claim 6, **characterized in that** the molar ratio of the dissolved compound of formula 1 in step a) to the fumaric acid in step b) is 1:0.6 to 1:1.3.

8. Method according to Claim 6 or 7, **characterized in that** the fumaric acid in step b) is added as solid to the solution in step a), or the fumaric acid in step b) is added as a solution in a suitable solvent to the solution in step a).

9. Method according to at least one of Claims 6 to 8 for producing form A, where the crystalline compound of formula 1 (Sitagliptin) and fumaric acid are stirred into 2-butanone (methyl ethyl ketone) and the molar ratio of Sitagliptin to fumaric acid is preferably 1:1 ± 0.1.

## Revendications

1. Composé cristallin de 7-[(3R)-3-amino-1-oxo-4-(2,4,5-trifluorophényl)butyl]-5,6,7,8-tétrahydro-3-(trifluorométhyl)-1,2,4-triazolo[4,3-a]pyrazine (INN : sitagliptine) de formule 1 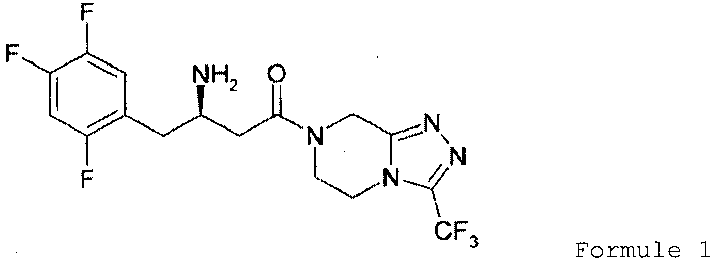 avec de l'acide fumarique, le rapport molaire entre le composé de formule 1 et l'acide fumarique étant de 1:0,95 à 1:1,05 et le composé présentant par diffractométrie de rayons X sur poudre (XRPD) des valeurs d (Å) à 12,9 (w), 10,3 (w), 5,15 (m) et 4,30 (vs), qui est désigné ultérieurement en tant que forme A.

2. Composé cristallin selon la revendication 1, **caractérisé en ce que** le composé présente par diffractométrie de rayons X sur poudre (XRPD) des valeurs d (Å) à 12,9 (w), 10,3 (w), 5,15 (m), 4,69 (m), 4, 30 (vs), 3, 50 (s), 3,22 (s).

3. Composé cristallin selon au moins l'une quelconque des revendications 1 et 2, destiné à une utilisation pour le traitement du diabète.

4. Médicament contenant le composé cristallin selon au moins l'une quelconque des revendications précédentes.

5. Utilisation du composé cristallin selon au moins l'une quelconque des revendications 1 à 3 pour la fabrication d'un médicament pour le traitement du diabète.

6. Procédé de fabrication du composé selon au moins l'une quelconque des revendications 1 à 2 comprenant les étapes suivantes :
a) la préparation d'une solution de 7-[(3R)-3-amino-1-oxo-4-(2,4,5-trifluorophényl)butyl]-5,6,7,8-tétrahydro-3-(trifluorométhyl)-1,2,4-triazolo[4,3-a]pyrazine (INN : sitagliptine) de formule 1,
b) l'ajout d'acide fumarique à la solution de l'étape a),
c) éventuellement la concentration de la composition obtenue à l'étape b) et/ou éventuellement l'ajout d'un non-solvant approprié pour réduire la solubilité du composé cristallin, et
d) la séparation du solide obtenu.

7. Procédé selon la revendication 6, **caractérisé en ce que** le rapport molaire entre le composé dissous de formule 1 à l'étape a) et l'acide fumarique à l'étape b) est de 1:0,6 à 1:1,3.

8. Procédé selon la revendication 6 ou 7, **caractérisé en ce que** l'acide fumarique est ajouté à l'étape b) sous forme solide à la solution de l'étape a) ou l'acide fumarique de l'étape b) est ajouté sous la forme d'une solution dans un solvant approprié à la solution de l'étape a).

9. Procédé selon au moins l'une quelconque des revendications 6 à 8 pour la fabrication de la forme A, selon lequel le composé cristallin de formule 1 (sitagliptine) et l'acide fumarique sont agités dans de la 2-butanone (méthyléthylcétone) et le rapport molaire entre la sitagliptine et l'acide fumarique est de préférence de 1:1 ± 0,1.
